# EUROPÄISCHE PATENTSCHRIFT

(11) **EP 2 272 863 B1**
(45) Veröffentlichungstag und Bekanntmachung des Hinweises auf die Patenterteilung: **31.10.2012**
(21) Anmeldenummer: 10009855.7
(22) Anmeldetag: 06.05.2004
(51) Int. Cl.: C07K 14/415, C12N 15/82

(54) **Poa p 5-Derivate mit reduzierter Allergenität und erhaltener T-Zellrealktivität**
Poa p 5-derivatives with reduced allergenicity and conserved T-cell reactivity
Dérivés de poa p 5 doté d'un effet allergénique réduit et d'une réactivité aux lymphocytes T conservée

(30) Priorität: 04.06.2003 DE 10325508
(43) Veröffentlichungstag der Anmeldung: 12.01.2011
(62) Teilanmeldung aus: 04739147.9
(73) Patentinhaber: Merck Patent GmbH, 64293 Darmstadt (DE)
(72) Erfinder: Wald, Martin, Dr., 20253 Hamburg (DE); Cromwell, Oliver, Dr., 23701 Suesel-Fassendorf (DE); Nandy, Andreas, Dr., 22175 Hamburg (DE); Kahlert, Helga, Dr., 22041 Hamburg (DE); Weber, Bernhard, Dr., 21031 Hamburg (DE); Fiebig, Helmut, Prof., 21493 Schwarzenbek (DE)

(56) Entgegenhaltungen:
- WO-A-03/025009
- SCHRAMM G ET AL: "Allergen engineering: variants of the timothy grass pollen allergen Phl p 5b with reduced IgE-binding capacity but conserved T cell reactivity", 15. Februar 1999 (1999-02-15), JOURNAL OF IMMUNOLOGY, WILLIAMS & WILKINS CO, US, PAGE(S) 2406-2414, XP002216586, ISSN: 0022-1767 * Discussion, Tabelle IV *

### Sequenz-Protokoll

<110> Merck Patent GmbH
<120> Phl p 5a-Derivate mit reduzierter Allergenität und erhaltener T-Zellreaktivität
<130> P 03/109
<140> DE 10325508.7
   <141> 2003-06-04
<160> 14
<170> PatentIn version 3.1
<210> 1
   <211> 855
   <212> DNA
   <213> Phleum pratense
<400> 1
   gccgatctag gctacggccc ggccacccca gctgccccgg ccgccggcta cacccccgcc     60
   gccccggccg gagcggagcc agcaggtaag gcgacgaccg aggagcagaa gctgatcgag    120
   aagatcaacg ccggcttcaa ggcggccttg gccgctgccg ccggcgtccc gccagcggac    180
   aagtacagga cgttcgtcgc aaccttcggc gcggcctcca acaaggcctt cgcggagggc    240
   ctctcgggcg agcccaaggg cgccgccgaa tccagctcca aggccgcgct cacctccaag    300
   ctcgacgccg cctacaagct cgcctacaag acagccgagg gcgcgacgcc tgaggccaag    360
   tacgacgcct acgtcgccac cctaagcgag gcgctccgca tcatcgccgg caccctcgag    420
   gtccacgccg tcaagcccgc ggccgaggag gtcaaggtta tccctgccgg cgagctgcag    480
   gtcatcgaga aggtcgacgc cgccttcaag gtcgctgcca ccgccgccaa cgccgcgccc    540
   gccaacgaca agttcaccgt cttcgaggcc gccttcaaca acgccatcaa ggcgagcacg    600
   ggcggcgcct acgagagcta caagttcatc cccgccctgg aggccgccgt caagcaggcc    660
   tacgccgcca ccgtcgccac cgcgccggag gtcaagtaca ccgtctttga gaccgcgctg    720
   aaaaaggcca tcaccgccat gtccgaggcc cagaaggctg ccaagcccgc tgccgctgcc    780
   accgccaccg caacctccgc cgttggcgcg gccaccggcg ccgccaccgc cgctactggt    840
   ggctacaaag tctga                                                     855
<210> 2
   <211> 284
   <212> PRT
   <213> Phleum pratense
<400> 2
   Ala Asp Leu Gly Tyr Gly Pro Ala Thr Pro Ala Ala Pro Ala Ala Gly
   1               5                   10                  15
   Tyr Thr Pro Ala Ala Pro Ala Gly Ala Glu Pro Ala Gly Lys Ala Thr
               20                  25                  30
   Thr Glu Glu Gln Lys Leu Ile Glu Lys Ile Asn Ala Gly Phe Lys Ala
           35                  40                  45
   Ala Leu Ala Ala Ala Ala Gly Val Pro Pro Ala Asp Lys Tyr Arg Thr
       50                  55                  60
   Phe Val Ala Thr Phe Gly Ala Ala Ser Asn Lys Ala Phe Ala Glu Gly
   65                  70                  75                  80
   Leu Ser Gly Glu Pro Lys Gly Ala Ala Glu Ser Ser Ser Lys Ala Ala
                   85                  90                  95
   Leu Thr Ser Lys Leu Asp Ala Ala Tyr Lys Leu Ala Tyr Lys Thr Ala
               100                 105                 110
   Glu Gly Ala Thr Pro Glu Ala Lys Tyr Asp Ala Tyr Val Ala Thr Leu
           115                 120                 125
   Ser Glu Ala Leu Arg Ile Ile Ala Gly Thr Leu Glu Val His Ala Val
       130                 135                 140
   Lys Pro Ala Ala Glu Glu Val Lys Val Ile Pro Ala Gly Glu Leu Gln
   145 150 155 160
   Val Ile Glu Lys Val Asp Ala Ala Phe Lys Val Ala Ala Thr Ala Ala
   165 170 175
   Asn Ala Ala Pro Ala Asn Asp Lys Phe Thr Val Phe Glu Ala Ala Phe
   180 185 190
   Asn Asn Ala Ile Lys Ala Ser Thr Gly Gly Ala Tyr Glu Ser Tyr Lys
   195 200 205
   Phe Ile Pro Ala Leu Glu Ala Ala Val Lys Gln Ala Tyr Ala Ala Thr
   210 215 220
   Val Ala Thr Ala Pro Glu Val Lys Tyr Thr Val Phe Glu Thr Ala Leu
   225 230 235 240
   Lys Lys Ala Ile Thr Ala Met Ser Glu Ala Gln Lys Ala Ala Lys Pro
   245 250 255
   Ala Ala Ala Ala Thr Ala Thr Ala Thr Ser Ala Val Gly Ala Ala Thr
   260 265 270
   Gly Ala Ala Thr Ala Ala Thr Gly Gly Tyr Lys Val
   275 280
<210> 3
   <211> 795
   <212> DNA
   <213> Phleum pratense
<400> 3
   gccgatctag gctacggccc ggccacccca gctgccccgg ccgccggcta cacccccgcc 60
   gccccggccg gagcggagcc agcaggtaag gcgacgaccg aggagcagaa gctgatcgag 120
   aagatcaacg ccggcttcaa ggcggccttg gccgctgccg ccggcgtccc gccagcggac 180
   aagtacagga cgttcgtcgc aaccttcggc gcggcctcca acaaggcctt cgcggagggc 240
   ctctcgggcg agcccaaggg cgccgccgaa tccagctccg gcgcgacgcc tgaggccaag 300
   tacgacgcct acgtcgccac cctaagcgag gcgctccgca tcatcgccgg caccctcgag 360
   gtccacgccg tcaagcccgc ggccgaggag gtcaaggtta tccctgccgg cgagctgcag 420
   gtcatcgaga aggtcgacgc cgccttcaag gtcgctgcca ccgccgccaa cgccgcgccc 480
   gccaacgaca agttcaccgt cttcgaggcc gccttcaaca acgccatcaa ggcgagcacg 540
   ggcggcgcct acgagagcta caagttcatc cccgccctgg aggccgccgt caagcaggcc 600
   tacgccgcca ccgtcgccac cgcgccggag gtcaagtaca ccgtctttga gaccgcgctg 660
   aaaaaggcca tcaccgccat gtccgaggcc cagaaggctg ccaagcccgc tgccgctgcc 720
   accgccaccg caacctccgc cgttggcgcg gccaccggcg ccgccaccgc cgctactggt 780
   ggctacaaag tctga 795
<210> 4
   <211> 264
   <212> PRT
   <213> Phleum pratense
<400> 4
   Ala Asp Leu Gly Tyr Gly Pro Ala Thr Pro Ala Ala Pro Ala Ala Gly
   1               5                   10                  15
   Tyr Thr Pro Ala Ala Pro Ala Gly Ala Glu Pro Ala Gly Lys Ala Thr
               20                  25                  30
   Thr Glu Glu Gln Lys Leu Ile Glu Lys Ile Asn Ala Gly Phe Lys Ala
           35                  40                  45
   Ala Leu Ala Ala Ala Ala Gly Val Pro Pro Ala Asp Lys Tyr Arg Thr
       50                  55                  60
   Phe Val Ala Thr Phe Gly Ala Ala Ser Asn Lys Ala Phe Ala Glu Gly
   65                  70                  75                  80
   Leu Ser Gly Glu Pro Lys Gly Ala Ala Glu Ser Ser Ser Gly Ala Thr
                   85                  90                  95
   Pro Glu Ala Lys Tyr Asp Ala Tyr Val Ala Thr Leu Ser Glu Ala Leu
               100                 105                 110
   Arg Ile Ile Ala Gly Thr Leu Glu Val His Ala Val Lys Pro Ala Ala
           115                 120                 125
   Glu Glu Val Lys Val Ile Pro Ala Gly Glu Leu Gln Val Ile Glu Lys
       130                 135                 140
   Val Asp Ala Ala Phe Lys Val Ala Ala Thr Ala Ala Asn Ala Ala Pro
   145 150 155 160
   Ala Asn Asp Lys Phe Thr Val Phe Glu Ala Ala Phe Asn Asn Ala Ile
   165 170 175
   Lys Ala Ser Thr Gly Gly Ala Tyr Glu Ser Tyr Lys Phe Ile Pro Ala
   180 185 190
   Leu Glu Ala Ala Val Lys Gln Ala Tyr Ala Ala Thr Val Ala Thr Ala
   195 200 205
   Pro Glu Val Lys Tyr Thr Val Phe Glu Thr Ala Leu Lys Lys Ala Ile
   210 215 220
   Thr Ala Met Ser Glu Ala Gln Lys Ala Ala Lys Pro Ala Ala Ala Ala
   225 230 235 240
   Thr Ala Thr Ala Thr Ser Ala Val Gly Ala Ala Thr Gly Ala Ala Thr
   245 250 255
   Ala Ala Thr Gly Gly Tyr Lys Val
   260
<210> 5
   <211> 783
   <212> DNA
   <213> Phleum pratense
<400> 5
   gccgatctag gctacggccc ggccacccca gctgccccgg ccgccggcta cacccccgcc 60
   gccccggccg gagcggagcc agcaggtaag gcgacgaccg aggagcagaa gctgatcgag 120
   aagatcaacg ccggcttcaa ggcggccttg gccgctgccg ccggcgtccc gccagcggac 180
   aagtacagga cgttcgtcgc aaccttcggc gcggcctcca acaaggcctt cgcggagggc 240
   ctctcgggcg agcccaaggg cgccgccgaa tccagctcca aggccgcgct cacctccaag 300
   ctcgacgccg cctacaagct cgcctacaag acagccgagg gcgcgacgcc tgaggccaag 360
   tacgacgcct acgtcgccac cctaagcgag gcgctccgca tcatcgccgg caccctcgag 420
   gtccacgccg tcaagcccgc ggccgaggag gtcaaggtta tccctgccgg cgagctgcag 480
   gtcatcgaga aggtcgacgc cgccttcaag gtcgctgcca ccagcacggg cggcgcctac 540
   gagagctaca agttcatccc cgccctggag gccgccgtca agcaggccta cgccgccacc 600
   gtcgccaccg cgccggaggt caagtacacc gtctttgaga ccgcgctgaa aaaggccatc    660
   accgccatgt ccgaggccca gaaggctgcc aagcccgctg ccgctgccac cgccaccgca    720
   acctccgccg ttggcgcggc caccggcgcc gccaccgccg ctactggtgg ctacaaagtc    780
   tga                                                                  783
<210> 6
   <211> 260
   <212> PRT
   <213> Phleum pratense
<400> 6
   Ala Asp Leu Gly Tyr Gly Pro Ala Thr Pro Ala Ala Pro Ala Ala Gly
   1               5                   10                  15
   Tyr Thr Pro Ala Ala Pro Ala Gly Ala Glu Pro Ala Gly Lys Ala Thr
               20                  25                  30
   Thr Glu Glu Gln Lys Leu Ile Glu Lys Ile Asn Ala Gly Phe Lys Ala
           35                  40                  45
   Ala Leu Ala Ala Ala Ala Gly Val Pro Pro Ala Asp Lys Tyr Arg Thr
       50                  55                  60
   Phe Val Ala Thr Phe Gly Ala Ala Ser Asn Lys Ala Phe Ala Glu Gly
   65                  70                  75                  80
   Leu Ser Gly Glu Pro Lys Gly Ala Ala Glu Ser Ser Ser Lys Ala Ala
                   85                  90                  95
   Leu Thr Ser Lys Leu Asp Ala Ala Tyr Lys Leu Ala Tyr Lys Thr Ala
               100                 105                 110
   Glu Gly Ala Thr Pro Glu Ala Lys Tyr Asp Ala Tyr Val Ala Thr Leu
           115                 120                 125
   Ser Glu Ala Leu Arg Ile Ile Ala Gly Thr Leu Glu Val His Ala Val
       130                 135                 140
   Lys Pro Ala Ala Glu Glu Val Lys Val Ile Pro Ala Gly Glu Leu Gln
   145                 150                 155                 160
   Val Ile Glu Lys Val Asp Ala Ala Phe Lys Val Ala Ala Thr Ser Thr
                   165                 170                 175
   Gly Gly Ala Tyr Glu Ser Tyr Lys Phe Ile Pro Ala Leu Glu Ala Ala
               180                 185                 190
   Val Lys Gln Ala Tyr Ala Ala Thr Val Ala Thr Ala Pro Glu Val Lys
           195                 200                 205
   Tyr Thr Val Phe Glu Thr Ala Leu Lys Lys Ala Ile Thr Ala Met Ser
       210                 215                 220
   Glu Ala Gln Lys Ala Ala Lys Pro Ala Ala Ala Ala Thr Ala Thr Ala
   225                 230                 235                 240
   Thr Ser Ala Val Gly Ala Ala Thr Gly Ala Ala Thr Ala Ala Thr Gly
                   245                 250                 255
   Gly Tyr Lys Val
               260
<210> 7
   <211> 723
   <212> DNA
   <213> Phleum pratense
<400> 7
   gccgatctag gctacggccc ggccacccca gctgccccgg ccgccggcta cacccccgcc      60
   gccccggccg gagcggagcc agcaggtaag gcgacgaccg aggagcagaa gctgatcgag     120
   aagatcaacg ccggcttcaa ggcggccttg gccgctgccg ccggcgtccc gccagcggac     180
   aagtacagga cgttcgtcgc aaccttcggc gcggcctcca acaaggcctt cgcggagggc     240
   ctctcgggcg agcccaaggg cgccgccgaa tccagctccg gcgcgacgcc tgaggccaag     300
   tacgacgcct acgtcgccac cctaagcgag gcgctccgca tcatcgccgg caccctcgag     360
   gtccacgccg tcaagcccgc ggccgaggag gtcaaggtta tccctgccgg cgagctgcag     420
   gtcatcgaga aggtcgacgc cgccttcaag gtcgctgcca ccagcacggg cggcgcctac     480
   gagagctaca agttcatccc cgccctggag gccgccgtca agcaggccta cgccgccacc     540
   gtcgccaccg cgccggaggt caagtacacc gtctttgaga ccgcgctgaa aaaggccatc     600
   accgccatgt ccgaggccca gaaggctgcc aagcccgctg ccgctgccac cgccaccgca     660
   acctccgccg ttggcgcggc caccggcgcc gccaccgccg ctactggtgg ctacaaagtc     720
   tga                                                                   723
<210> 8
   <211> 240
   <212> PRT
   <213> Phleum pratense
<400> 8
   Ala Asp Leu Gly Tyr Gly Pro Ala Thr Pro Ala Ala Pro Ala Ala Gly
   1               5                   10                  15
   Tyr Thr Pro Ala Ala Pro Ala Gly Ala Glu Pro Ala Gly Lys Ala Thr
               20                  25                  30
   Thr Glu Glu Gln Lys Leu Ile Glu Lys Ile Asn Ala Gly Phe Lys Ala
           35                  40                  45
   Ala Leu Ala Ala Ala Ala Gly Val Pro Pro Ala Asp Lys Tyr Arg Thr
       50                  55                  60
   Phe Val Ala Thr Phe Gly Ala Ala Ser Asn Lys Ala Phe Ala Glu Gly
   65                  70                  75                  80
   Leu Ser Gly Glu Pro Lys Gly Ala Ala Glu Ser Ser Ser Gly Ala Thr
                   85                  90                  95
   Pro Glu Ala Lys Tyr Asp Ala Tyr Val Ala Thr Leu Ser Glu Ala Leu
               100                 105                 110
   Arg Ile Ile Ala Gly Thr Leu Glu Val His Ala Val Lys Pro Ala Ala
           115                 120                 125
   Glu Glu Val Lys Val Ile Pro Ala Gly Glu Leu Gln Val Ile Glu Lys
       130                 135                 140
   Val Asp Ala Ala Phe Lys Val Ala Ala Thr Ser Thr Gly Gly Ala Tyr
   145                 150                 155                 160
   Glu Ser Tyr Lys Phe Ile Pro Ala Leu Glu Ala Ala Val Lys Gln Ala
                   165                 170                 175
   Tyr Ala Ala Thr Val Ala Thr Ala Pro Glu Val Lys Tyr Thr Val Phe
               180                 185                 190
   Glu Thr Ala Leu Lys Lys Ala Ile Thr Ala Met Ser Glu Ala Gln Lys
           195                 200                 205
   Ala Ala Lys Pro Ala Ala Ala Ala Thr Ala Thr Ala Thr Ser Ala Val
       210                 215                 220
   Gly Ala Ala Thr Gly Ala Ala Thr Ala Ala Thr Gly Gly Tyr Lys Val
   225                 230                 235                 240
<210> 9
   <211> 24
   <212> DNA
   <213> Phleum pratense
<400> 9
   gccgatctag gctacggccc ggcc 24
<210> 10
   <211> 36
   <212> DNA
   <213> Phleum pratense
<400> 10
   aacataacta gtggcagcga ccttgaaggc ggcgtc 36
<210> 11
   <211> 30
   <212> DNA
   <213> Phleum pratense
<400> 11
   atctaactag tacgggcggc gcctacgaga 30
<210> 12
   <211> 33
   <212> DNA
   <213> Phleum pratense
<400> 12
   aacataaagc tttcagactt tgtagccacc agt 33
<210> 13
   <211> 27
   <212> DNA
   <213> Phleum pratense
<400> 13
   ggagctggat tcggcggcgc ccttggg 27
<210> 14
   <211> 45
   <212> DNA
   <213> Phleum pratense
<400> 14
   gccgccgaat ccagctccgg cgcgacgcct gaggccaagt acgac 45

## Beschreibung

Die vorliegende Erfindung betrifft die Herstellung und Verwendung von Varianten des Gruppe-5-Allergens Poa p 5 aus *Poa pratensis,* welche eine gegenüber dem bekannten Wildtyp-Allergen verringerte IgE-Reaktivität sowie eine weitgehend erhaltene Reaktivität mit T-Lymphozyten aufweisen, dadurch gekennzeichnet, dass ein Bereich, der dem Aminosäuresequenz-Bereich 94 -113 des Phl p 5a entspricht, oder die Bereiche, die den Aminosäuresequenz-Bereichen 94 - 113 und 175 - 198 des Phl p 5a entsprechen, im Vergleich zu dem bekannten Wildtyp-Allergen fehlen.
Diese hypoallergenen Allergenvarianten können zur spezifischen Immuntherapie (Hyposensibilisierung) von Patienten mit Graspollenallergie oder zur präventiven Immuntherapie von Graspollenallergien eingesetzt werden.

### Hintergrund der Erfindung

Allergien vom Typ 1 haben weltweite Bedeutung. Bis zu 20 % der Bevölkerung in industrialisierten Ländern leiden unter Beschwerden wie allergischer Rhinitis, Konjunktivitis oder Bronchialasthma. Diese Allergien werden durch in der Luft befindliche Allergene (Aeroallergene), die von Quellen unterschiedlicher Herkunft wie Pflanzenpollen, Milben, Katzen oder Hunden freigesetzt werden, hervorgerufen. Bis zu 40 % dieser Typ 1-Allergiker wiederum zeigen spezifische IgE-Reaktivität mit Gräserpollenallergenen (Freidhoff et al., 1986, J. Allergy Clin. Immunol. 78, 1190-2002).

Bei den Typ 1-Allergie auslösenden Substanzen handelt es sich um Proteine, Glykoproteine oder Polypeptide. Diese Allergene reagieren nach Aufnahme über die Schleimhäute mit den bei sensibilisierten Personen an der Oberfläche von Mastzellen gebundenen IgE-Molekülen. Werden zwei IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden klinischen Symptomen.

In Abhängigkeit von der relativen Häufigkeit mit der die einzelnen Allergenmoleküle mit den IgE-Antikörpern von Allergikern reagieren, wird zwischen Major- und Minorallergenen unterschieden.

Im Fall von Wiesenlieschengras (*Phleum pratense)* sind bislang Phl p 1 (Petersen et al., 1993, J. Allergy Clin. Immunol. 92: 789-796), Phl p 5 (Matthiesen und Löwenstein, 1991, Clin. Exp. Allergy 21: 297-307; Petersen et al., 1992, Int. Arch. Allergy Immunol. 98: 105-109), Phl p 6 (Petersen et al., 1995, Int. Arch. Allergy Immunol. 108, 49-54). Phl p 2/3 (Dolecek et al., 1993, FEBS 335 (3), 299-304), Phl p 4 (Haavik et al., 1985, Int. Arch. Allergy Appl. Immunol. 78: 260-268; Valenta et al., 1992, Int. Arch. Allergy Immunol. 97: 287-294, Fischer et al., 1996, J. Allergy Clin. Immunol. 98: 189-198) und Phl p 13 (Suck et al., 2000, Clin. Exp. Allergy 30: 324-332; Suck et al., 2000, Clin. Exp. Allergy 30: 1395-1402) als Hauptallergene identifiziert worden.

Die dominierenden Hauptallergene des Wiesenlieschgrases *(Phleum pratense)* sind Phl p 1 und Phl p 5, wobei Phl p 5 in zwei hinsichtlich ihrer Molekularmasse differenten Formen 5a und 5b, die von unabhängigen Genen kodiert werden, vorkommt. Die deduzierten Aminosäuresequenzen sowohl von Phl p 5a als auch von Phl p 5b konnten mittels rekombinanter DNA - Technik bestimmt werden. Phl p 5a ist ein Protein von ca. 32 kDa und reagiert mit den IgE-Antikörpern von 85 - 90 % der Graspollenallergiker. Phl p 5a existiert in einer Serie von homologen Varianten, die sich durch Punktmutationen voneinander unterscheiden und wahrscheinlich unterschiedlichen allelen Formen entsprechen. Die Pollen der verwandten Grasspezies wie z. B. *Lolium perenne, Poa pratensis* u. a. enthalten Allergene, die dem Phl p 5a homolog sind und zusammen als Gruppe 5-Allergene bezeichnet werden. Die hohe strukturelle Homologie dieser Gruppe 5-Allergene der Gräserarten bedingt eine entsprechend hohe Kreuzreaktivität der Moleküle mit den IgE-Antikörpern von Graspollenallergikern.

Ein klassischer Ansatz zur wirksamen therapeutischen Behandlung von Allergien stellt die spezifische Immuntherapie oder Hyposensibilisierung dar (Fiebig, 1995, Allergo J. 4 (6): 336-339, Bousquet et al., 1998, J. Allergy Clin. Immunol. 102 (4): 558-562). Dabei werden dem Patienten natürliche Allergenextrakte in steigenden Dosen subkutan injiziert. Allerdings besteht bei dieser Methode die Gefahr von allergischen Reaktionen oder sogar eines anaphylaktischen Schocks. Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig, 1995, Allergo J. 4 (7): 377-382).

Eine noch weitergehende Therapieoptimierung wäre mit rekombinant hergestellten Allergenen möglich. Definierte, ggfs. auf die individuellen Sensibilierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten.
Realistische Perspektiven, die zu einer sicheren Hyposensibilierung mit rekombinanten Expressionsprodukten führen können, bieten gezielt mutierte rekombinante Allergene, bei denen IgE-Epitope spezifisch deletiert werden, ohne die für die Therapie essentiellen T-Zell Epitope zu beeinträchtigen (Schramm et al., 1999, J. Immunol. 162: 2406-2414).
Eine weitere Möglichkeit zur therapeutischen Beeinflussung des gestörten T-Helferzellen-Gleichgewichtes bei Allergikern ist die Behandlung mit expressionsfähiger DNA, die für die relevanten Allergene kodiert (immuntherapeutische DNA-Vakzinierung). Erste experimentelle Belege für die allergenspezifische Beeinflussung der Immunantwort durch eine solche DNA-Vakzine konnte an Nagern durch Injektion von Allergen-kodierender DNA erbracht werden (Hsu et al., 1996, Nature Medicine 2 (5): 540-544).

Die der vorliegenden Erfindung zugrund liegende Aufgabe bestand nun in der Bereitstellung neuer Varianten des Gruppe-5-Allergens Poa p 5 auf Protein- und DNA-Ebene, die sich bei weitgehendem Erhalt der T-Zell-Reaktivität durch eine reduzierte IgE-Aktivität auszeichnen und daher für die spezifische Immuntherapie sowie die immuntherapeutische DNA-Vakzinierung geeignet sind.

### Abbildungen

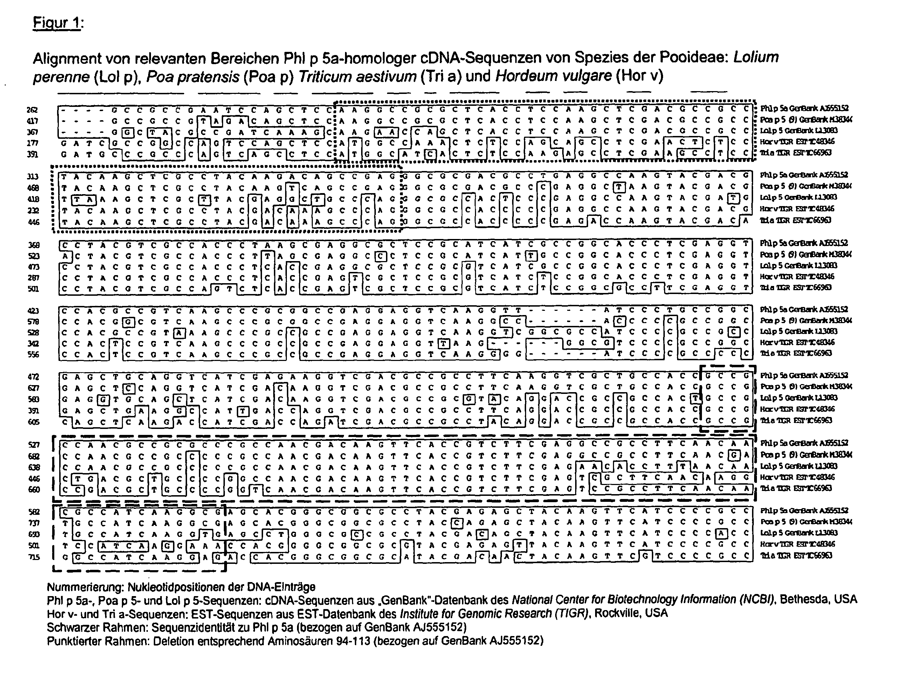

**Abbildung 1****:** Alignment von relevanten Bereichen Phl p 5a-homologer cDNA-Sequenzen von Spezies der *Pooideae: Lolium perenne* (Lol p), *Poa pratensis* (Poa p) *Triticum aestivum* (Tri a) und *Hordeum vulgare* (Hor v) Nummerierung: Nukleotidpositionen der DNA-Einträge
   Phl p 5a-, Poa p 5- und Lol p 5-Sequenzen: cDNA-Sequenzen aus "GenBank"-Datenbank des National Center for Biotechnology Information (NCBI), Bethesda, USA
   Hor v- und Tri α-Sequenzen: EST-Sequenzen aus EST-Datenbank des Institute for Genomic Research (TIGR), Rockville, USA
   Schwarzer Rahmen: Sequenzidentität zu Phl p 5a (bezogen auf GenBank AJ555152)
   Punktierter Rahmen: Deletion entsprechend Aminosäuren 94-113 (bezogen auf GenBank AJ555152)
   Gestrichelter Rahmen: Deletion entsprechend Aminosäuren 175-198 (bezogen auf GenBank AJ555152)

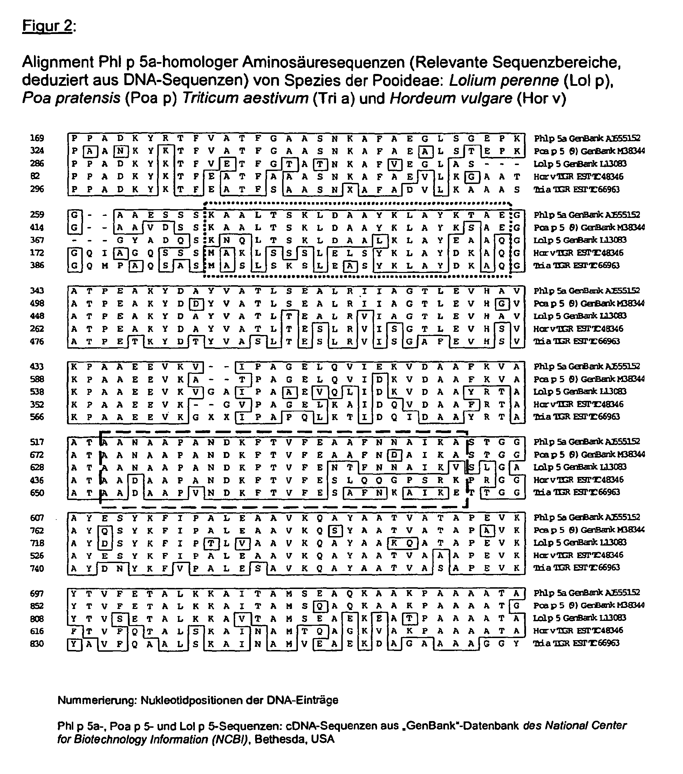

**Abbildung 2****:** Alignment Phl p 5a-homologer Aminosäuresequenzen (Relevante Sequenzbereiche, deduziert aus DNA-Sequenzen) von Spezies der *Pooideae: Lolium perenne* (Lol p), *Poa pratensis* (Poa p) *Triticum aestivum* (Tri a) und *Hordeum vulgare* (Hor v)
   Nummerierung: Nukleotidpositionen der DNA-Einträge
   Phl p 5a-, Poa p 5- und Lol p 5-Sequenzen: cDNA-Sequenzen aus "GenBank"-Datenbank des National Center for Biotechnology Information (NCBI), Bethesda, USA
   Hor v- und Tri α-Sequenzen: EST-Sequenzen aus EST-Datenbank des Institute for Genomic Research (TIGR), Rockville, USA
   Schwarzer Rahmen: Sequenzidentität zu Phl p 5a (bezogen auf GenBank AJ555152)
   Punktierter Rahmen: Deletion entsprechend Aminosäuren 94-113 (bezogen auf GenBank AJ555152)
   Gestrichelter Rahmen: Deletion entsprechend Aminosäuren 175-198 (bezogen auf GenBank AJ555152)
**Abbildung 3****:** SDS-PAGE von gereinigten Deletionsmutanten in Form von Histidin-Fusionsproteinen
   1) Marker
   2) rPhl p 5a wt (His)
   3) Phl p 5a DM-D94-113 (His)
   4) Phl p 5a DM-D94-113, 175-198 (His)
   5) Phl p 5a DM-D175-198 (His)
   6) Marker
**Abbildung 4****:** SDS-PAGE der gereinigten Nicht-Fusionsproteine Phl p 5a DM-D94-113, 175-198 und rPhl p 5a wt (oben) und Identitätstest mit aPhl p 5-Antikörpem (unten)
   Der aPhl p 5 mAb Apha-1D11 bindet die Region 175-198
   (nur rPhl p 5a wt ist positiv)
   Der aPhl p 5a mAb Apha-3B2 bindet ein gemeinsames
   Epitop der beiden Phl p 5a-Moleküle (beide Proteine positiv)
   (mAb: Monoklonaler Antikörper)
**Abbildung 5****:** Analytische SEC der Deletionsmutante Phl p 5a DM-D94-113, 175-198 und des rekombinanten Phl p 5a-Wildtypes (gereinigte Nicht-Fusionsproteine)
   Säule: Superdex 75 HR10/30 (Amersham Biosciences, Uppsala, Schweden)
   Laufmittel: PBS
   Pfeil: Ausschlussvolumen
**Abbildung 6****:** Nicht-denaturierende Isoelektrische Fokussierung der Deletionsmutante Phl p 5a DM-D94-113, 175-198 und des rekombinanten Phl p 5a-Wildtypes (gereinigte Nicht-Fusionsproteine)
   1) IEF-Marker
   2) rPhl p 5a wt
   3) Phl p 5a DM-D94-113, 175-198
   pl rPhl p 5a wt = 8.7
   pl rPhl p 5a DM-D94-113, 175-198 = 6.4
**Abbildung 7****:** Streifentest zur Überprüfung der IgE-Bindungsfähigkeit von Phl p 5a-Deletions-mutanten (nicht-denaturierend)
   P: Seren klinisch definierter Gräserpollen-Allergiker
**Abbildung 8****:** Nachweis der reduzierten IgE-Reaktivität der Phl p 5a-Deletionsmutanten mittels EAST-Inhibitionstest mit zwei repräsentativen Einzelseren (a und b) und einem Serum-Pool (c)
   -•- nPh p 5a/ b
      -○- rPhl p 5a wt
      -Δ- rPhl p 5a wt(His)
   -◊- Phl p 5a DM-Δ94-113 (His)
      -◊- Phl p 5a DM-Δ175-198 (His)
   -■- Phl p 5a DM-Δ94-113, 175-198
      -□- Phl p 5a DM-Δ94-113, 175-198 (His)
   P: Seren klinisch definierter Gräserpollen-Allergiker
**Abbildung 9****:** Nachweis der Hypoallergenität der Phl p 5a-Deletionsmutante Phl p 5a DM-D94-113, 175-198 mittels Basophilen-Aktivierungstest mit Basophilen von sechs verschiedenen Gräserpollen-Allergikern (P)

### Detaillierte Beschreibung der Erfindung

### Mutagenese und Klonierung von cDNA-Sequenzen

Ausgangspunkt für die - erfindungsgemäß besonders bevorzugten - hypoallergenen Phl p 5a-Varianten ist die cDNA einer Isoform des Phl p 5a Wildtyps, die mit Hilfe spezifischer Primer durch Polymerase-Kettenreaktion (PCR) aus der Gesamt-cDNA von Pollen des Wiesen-Lieschgrases (*Phleum pratense)* isoliert wurde (NCBI (National Centrer for Biotechnology Information, Betesda, USA) GenBank Nummer AJ555152) (SEQ ID NO 1). Von der cDNA-Sequenz konnte die Aminosäuresequenz gemäß SEQ ID NO 2 deduziert werden. Das aus 284 Aminosäuren bestehende Phl p 5a wurde in E. coli cytosolisch als lösliches Protein exprimiert und nachfolgend gereinigt. Diese rekombinante Wildtyp-Form von Phl p 5a (rPhl p 5a wt) reagiert mit monoklonalen anti-Phl p 5 Antikörpern und mit IgE-Antikörpern von Grasspollenallergikern, die eine Reaktivität mit natürlichem gereinigtem Phl p 5a (nPhl p 5a) aufwiesen.

Ausgehend von der beschriebenen cDNA von rPhl p 5a wt wurde eine Serie von differenten Deletionsvarianten (Deletionsmutanten) durch Restriktions-/ Ligationsverfahren und PCR hergestellt und in den Expressionsvektor pProExHTa (Invitrogen, Carlsbad, USA) ligiert. Es wurden Abschnitte von 6 bis 72 bp Länge verteilt über die ganze Sequenz des cDNA-Moleküls deletiert, wodurch entsprechende Deletionen in den Polypeptidketten der in E. coli exprimierten Proteine induziert wurden.

Die Deletionsvarianten von Phl p 5a wurden mittels Immunoblot auf ihre Bindungsfähigkeit an IgE-Antikörper eines repräsentativen Serumpools von Grasspollenallergikern untersucht.

Bei diesem Verfahren wurden überraschenderweise zwei Deletionsvarianten des Phl p 5a (Phl p 5a DM-Δ94-113, Deletion von Aminosäuren 94-113 und Phl p 5a DM-Δ175-198, Deletion von Aminosäuren 175-198 des rPhl p 5a wt) gefunden, die eine verminderte Bindung von IgE-Antikörpern (repräsentativer Serumpool) aufweisen.
Diese beiden Phl p 5a-Deletionen dienten als Ausgangspunkt für die Konstruktion einer Doppel-Deletionsmutante, die beide wirksamen Deletionen enthält (Phl p 5a DM -Δ94-113, 175-198).

Die gentechnische Konstruktion von Phl p 5a DM-Δ94-113, Phl p 5a DM-Δ175-198 und Phl p 5a DM-Δ94-113, 175-198 sowie deren biochemische und immunologische Charakterisierung sind im folgenden beschrieben.

Für die Konstruktion der Deletionsvariante Phl p 5a DM-Δ94-113 (SEQ ID NO 3, cDNA-Sequenz (795 bp), und SEQ ID NO 4, Aminosäuresequenz (264 aa)) wurden zunächst zwei Fragmente ausgehend von der cDNA von rPhl p 5a wt hergestellt. Das Fragment "F1-93", kodierend für Aminosäuren 1-93 von rPhl p 5a wt, wurde durch PCR mit Hilfe der Primer 1 und 5, und das Fragment "F114-284" mit Hilfe der Primer 4 und 6 hergestellt (Primersequenzen s. Tab. 1). Die Fragmente "F1-93" und "F114-284" wurden in eine weitere PCR unter Einsatz der Primer 1 und 4 als Matrize eingesetzt, was in der Amplifikation der vollständigen cDNA kodierend für die Deletionsvariante Phl p 5a DM-Δ94-113 resultierte. Grundlage der Verbindung der Fragmente "F1-93" und "F114-284" durch PCR war ein beiden Fragmenten gemeinsamer Sequenzbereich. Dieser Sequenzbereich entstand dadurch, dass Fragment "F114-284" durch PCR mittels eines besonderen sense-Oligonukleotides amplifiziert wurde, welches im 5'-Bereich eine zusätzliche DNA-Sequenz kodierend für Aminosäuren 88-93 enthielt (Tab. 1).

Die cDNA-Sequenz kodierend für die Deletionsvariante Phl p 5a DM-Δ175-198 (SEQ ID NO 5, cDNA-Sequenz (783 bp), und SEQ ID NO 6, Aminosäuresequenz (260 aa)) wurde durch Restriktion und anschliessende Ligation von zwei getrennt hergestellten cDNA-Fragmenten generiert. Das 5'-terminale Fragment "F1-174" wurde mithilfe von Primer 1 und 2 und das 3'-terminale Fragment "F199-284" mit Hilfe von Primer 3 und 4 durch PCR hergestellt. Die cDNA-Fragmente wurden mit dem Restriktionsenzym Spel verdaut und anschliessend ligiert (s. Tab. 1). Das Ligationsprodukt wurde unter Einsatz der Primern 1 und 4 durch PCR vermehrt.

Die cDNA der Deletionsvariante Phl p 5a DM-Δ94-113, 175-198 (SEQ ID NO 7, cDNA-Sequenz (723 bp), und SEQ ID NO 8, Aminosäuresequenz (240 aa)) wurde ebenfalls aus zwei cDNA-Fragmenten hergestellt. Das 5'-terminale Fragment wurde mit Primer 1 und 5 und mit rPhl p 5a wt-cDNA als Matrize, und das 3'-terminale Fragment mit Primer 4 und 6 mit Phl p 5a DM-Δ175-198-cDNA als Matrize generiert. Durch den gemeinsamen Sequenzbereich entsprechend Aminosäuren 88-93 des rPhl p 5a wt-Proteins wurden die Fragmente durch eine dritte PCR mit den Primern 1 und 4 verbunden, und das Produkt amplifiziert.
Die für die modifizierten Allergene kodierenden cDNA's wurden über die Restriktionsschnittstellen *Ehe*I und *Hind*III in den Expressionsvektor pProExHT (Invitrogen, Carlsbad, USA) ligiert, und anschliessend vollständig sequenziert.

Die immunologische Kreuzreaktivität der Gruppe 5-Allergene der *Pooideae, wie z.B. Poa pratensis und Lolium perenne,* basiert auf einer sehr ähnlichen Aminosäuresequenz. Es gilt als gesichert, dass die entsprechenden Gene auf ein gemeinsames Progenitor-Gen zurückgehen. Sowohl für die Sequenzen der Deletionen Δ94-113 und Δ175-198 der Phl p 5a wt-Proteinsequenz (Bezug: GenBank AJ555152) als auch für deren flankierenden Sequenzbereiche existieren homologe Sequenzbereiche in den Gruppe 5-Allergenen der *Pooideae.* Die hohe Homologie der betreffenden Sequenzbereiche ist sowohl auf der Ebene der DNA- als auch Aminosäuresequenz nachweisbar (Abb. 1 und Abb. 2).

**Tabelle 1: Aufstellung der zur Herstellung von Deletionsvarianten eingesetzten PCR-Primer**

| **Primer** | **SEQ ID NO** | **Richtung** | **Sequenz (5'→3')** |
|---|---|---|---|
| 1 | 9 | sense | gcc gat cta ggc tac ggc ccg gcc |
| 2 | 10 | antisense | |
| 3 | 11 | sense | atc ta act agt acg ggc ggc gcc tac gaga |
| 4 | 12 | antisense | aac ata aag ctt tca gac ttt gta gcc acc agt |
| 5 | 13 | antisense | gga gct gga ttc ggc ggc gcc ctt ggg |
| 6 | 14 | sense | |

Die *Spe*I-Restriktionsstellen sind durch Unterstreichung gekennzeichnet

### Expression und Reinigung rekombinanter PhI p 5a Moleküle

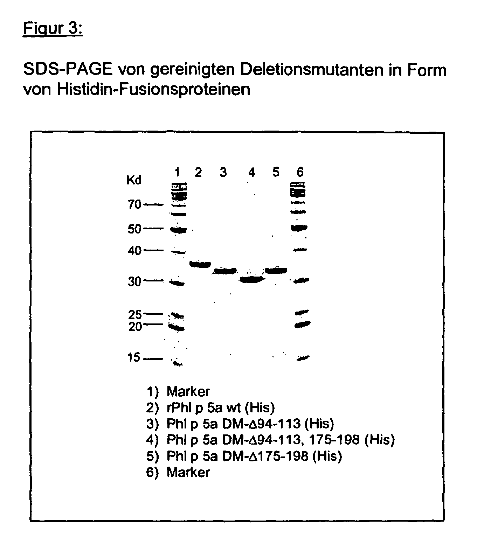

Die Expression der rekombinanten Proteine erfolgte als Histidin-Fusionsproteine mit integrierter Proteasespaltstelle (Expressionsvektor pProExHT; Invitrogen, Carlsbad, USA) zur optionalen Abspaltung des Histidin-Fusionsanteils (His) in *Escherichia coli* (Stamm JM109). rPhl p5a wt sowie die Deletionsmutanten wurden zunächst durch die spezifische Bindung der N-terminalen Histidinreste an eine Ni2+-Chelat-Matrix (Immobilized-Metal-lon-Affinity-Chromatography, IMAC) und nachfolgend durch präparative Gelfiltration (Size-Exclusion-Chromatography, SEC) gereinigt. Die Reinheit der eluierten Proteine wurde durch SDS-PAGE und analytische SEC kontrolliert. Die Ergebnisse zeigten, dass rPhl p 5a wt (His), Phl p 5a DM-Δ94-113 (His); Phl p 5a DM-Δ175-198 (His) und Phl p 5a DM-Δ94-113, 175-198 (His) mit hoher Reinheit und monomer dargestellt werden konnten (Abb. 3). Die Identität der Proteine wurde durch Phl p 5a-spezifische monoklonale Antikörper nachgewiesen.

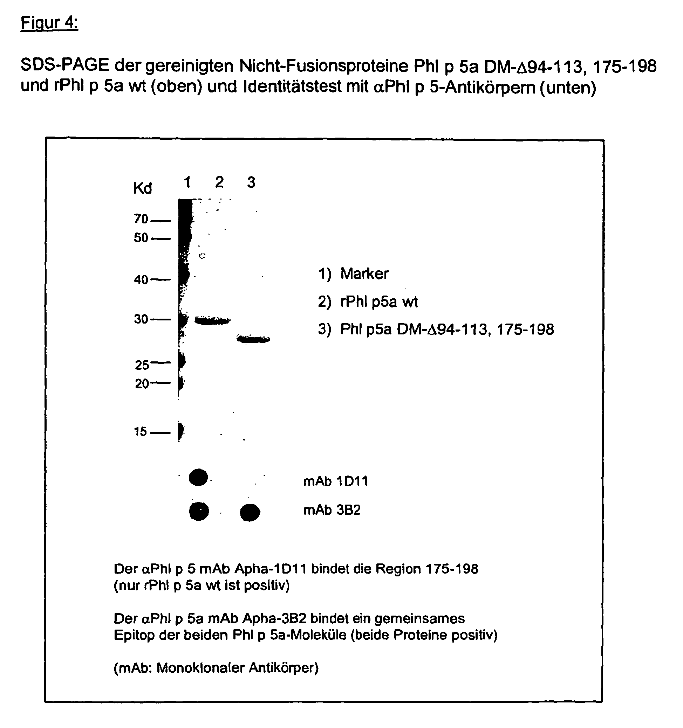

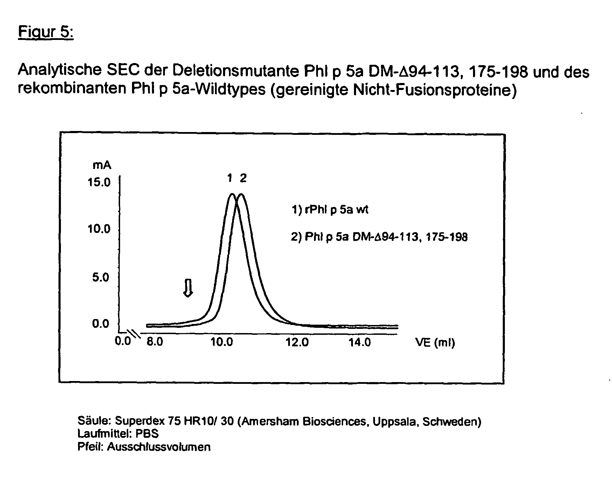

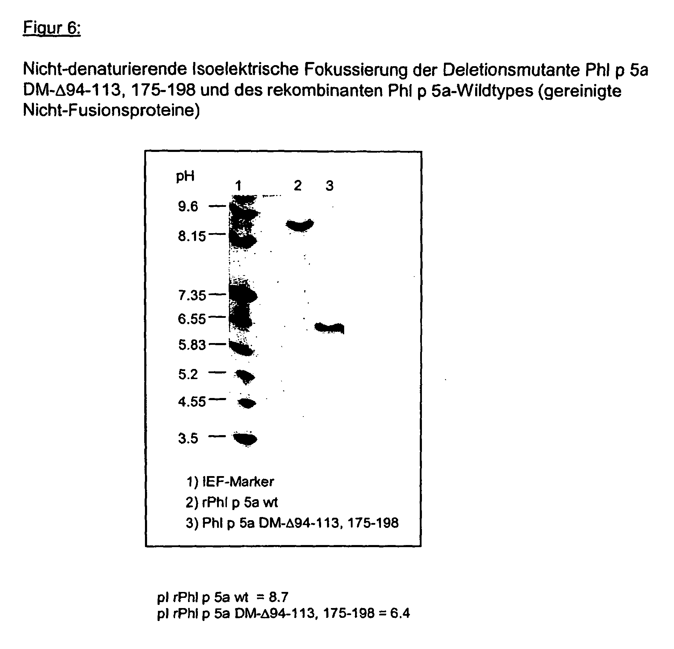

Die Überprüfung der IgE-Reaktivität mittels IgE-Bindungstechniken (Immunoblotting, Streifentest, EAST-Inhibitionstest und Basophilenaktivierungstest) sowie die Untersuchung der T-Zell-Reaktivität wurde außerdem mit Testsubstanzen ohne Histidin-Fusionsanteil durchgeführt.
Hierzu wurde die Deletionsvarianten parallel zu dem Vergleichsprotein rPhl p 5a-wt zunächst als Fusionsproteine hergestellt. Nachfolgend wurde jedoch der Histidin-Fusionsanteil enzymatisch abgespalten (TEV-Protease, Invitrogen, Carlsbad, USA), wodurch lediglich ein Glycin als Rest der Protease-Spaltsequenz an dem N-terminus des Zielprotein zurückblieb. Sowohl der abgespaltene Histidinanteil als auch die zur Spaltung verwendete Protease wurden durch IMAC vollständig abgetrennt. Nach einer präparativen SEC wurde die Reinheit und die Konformation der eluierten Proteine durch SDS-PAGE und analytische SEC überprüft, wie für rPhl p 5a wt und die Mutante Phl p 5a DM-Δ94-113, 175-198 in den Abbildungen 4 bzw. 5 dargestellt ist. Alle Proteine wurden rein und monomer dargestellt. Eine Untersuchung durch nicht-denaturierende Isoelektrische Fokussierung (IEF) der Nicht-Fusionsproteine zeigte stets eine hohe Homogenität hinsichtlich der Oberflächenladung (s. Abb. 6, beispielhaft für Phl p 5a DM-Δ94-113, 175-198).
Die Identität der rekombinanten Proteine wurde durch die monoklonalen Anti-Phl p 5-Antikörper (Allergopharma, Reinbek, Deutschland) Apha-1 D11 oder Apha-3B2 (s. Abb. 4, beispielhaft für Phl p 5a DM-Δ94-113, 175-198) und N-terminale Sequenzierung nachgewiesen.

### Nachweis der reduzierten IgE-Bindung der Phl p 5a-Deletionsvarianten

Eine einfacheTestmethode zur Bestimmung der IgE-Reaktivität von allergenen Molekülen ist die Untersuchung der Bindung von spezifischem IgE aus Allergikerseren an membrangebundene Testproteine durch den Streifentest.
Dafür werden die Testsubstanzen in gleicher Konzentration und Menge nebeneinander an einen Streifen von Nitrocellulose-Membran unter nichtdenaturierenden Bedingungen gebunden. Eine Reihe solcher Membranstreifen kann parallel mit unterschiedlichen Allergikerseren inkubiert werden. Nach einem Waschschritt werden die spezifisch gebundenen IgE-Antikörper durch eine Farbreaktion, vermittelt von einem Anti-hlgE/ Alkalische Phosphatase-Konjugat, auf der Membran sichtbar gemacht.

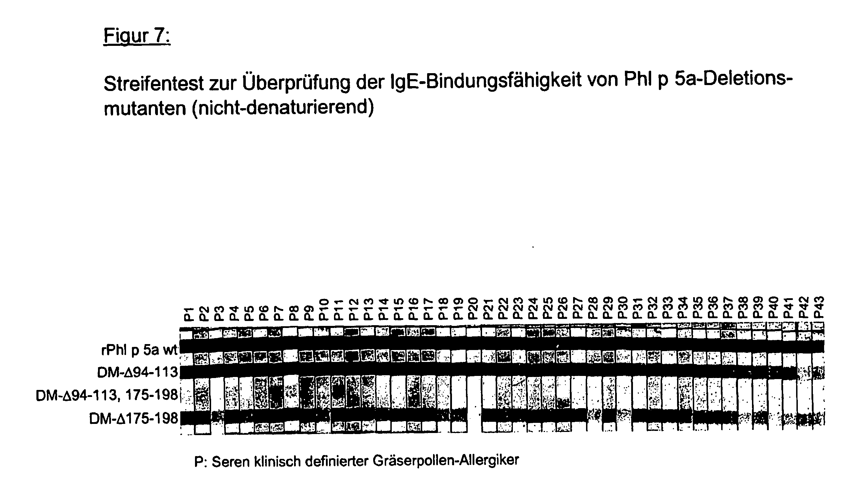

Die IgE-Reaktivität der rekombinanten Proteine Phl p 5a wt (His), Phl p 5a DM-Δ94-113 (His), Phl p 5a DM-Δ175-198 (His) und Phl p 5a DM-Δ94-113, 175-198 (His) wurde im Streifentest unter Einsatz von 43 individuellen Grasspollen-Allergikerseren vergleichend untersucht (Abb. 7).
Alle 43 Allergikerseren enthielten Phl p 5a-spezifische IgE-Antikörper, die mit dem natürlichen Phl p 5a (nPhl p 5a, hier nicht gezeigt) und dem rekombinanten Äquivalent rPhl p 5a wt (His) stark reagierten. Überraschenderweise wurde deutlich, dass die Phl p 5a-spezifischen IgE-Antikörper aller 43 Patientenseren gar nicht oder nur sehr stark vermindert an die Deletionsvariante Phl p 5a DM-Δ94-113, 175-198 (His) banden. Die reduzierte IgE-Bindung ist sowohl auf die Deletion Δ94-113 als auch auf die Deletion Δ175-198 zurückzuführen. Die Deletionsvariante Phl p 5a DM-Δ175-198 (His) zeigt bei diesem Test bei 35 von 43 Allergikerseren eine klar erkennbar verminderte IgE-Bindungsfähigkeit. In einigen Tests war der Einfluß der Deletion von Aminosäuren 175-198 so hoch, dass die IgE-Bindung fast vollständig verhindert wurde (Bsp.: P3, P20, P28)

Der Einfluß der Deletion Δ94-113 auf die IgE-Bindungsreaktivität ist weniger stark ausgeprägt, aber ebenfalls deutlich sichtbar. Die Deletionsvariante Phl p 5a DM-Δ94-113 (His) wurde von IgE von 19 der 43 individuellen Allergikerseren deutlich schwächer als die Referenz rPhl p 5a wt (His) gebunden (Bsp.: P31, P37, P42). Die Reduktion der IgE-Bindung war allerdings in vielen Einzeltests weniger drastisch ausgeprägt als die Reduktion verursacht durch Δ175-198.

Somit wird deutlich, dass beide Deletionen zu der Verminderung der Gesamt-IgE-Bindungsreaktivität der Deletionsmutante Phl p 5a DM-Δ94-113,175-198 (His) beitragen.

Im Gegensatz zu dem Streifentest können mit dem EAST-Hemmtest (Enzyme Allergosorbent Test) Allergen/ IgE-Interaktionen in Lösung untersucht werden, womit eine störende Maskierung von Epitopen der Testsubstanz durch die Immobilisierung an die Membran grundsätzlich ausgeschlossen werden kann. Der EAST-Hemmtest wird wie folgt ausgeführt. Mikrotiterplatten werden mit Allergenen, hier natürliches Phl p 5 (nPhl p 5a/b, Gemisch aus Phl p 5a und Phl p 5b), beschichtet. Nach Entfernung der nicht gebundenen Allergenmoleküle durch Waschen wird die Platte zur Vermeidung späterer unspezifischer Bindungen mit Rinderserumalbumin blockiert. IgE-Antikörper von Allergikern, als repräsentativer Pool von Einzelseren (Serum-Pool) oder als Einzelserum, wird in geeigneter Verdünnung mit den Allergen-beschichteten Mikrotiterplatten inkubiert. Die Menge der Allergengebundenen IgE-Antikörper wird über ein Zweitantikörper-gekoppeltes Enzym (Anti-hlgE/ Alkalische Phosphatase-Konjugat) durch die Umsetzung eines Substrates zu einem farbigen Endprodukt photometrisch quantifiziert. Die Bindung der IgE-Antikörper wird durch ein lösliches Allergen bzw. die zu prüfende Substanz (rekombinantes modifiziertes Allergen) in Abhängigkeit von der Konzentration substanzspezifisch gehemmt. Immunchemisch identische Substanzen zeigen identische Hemmkurven.

Als Referenzmoleküle dienten in dieser Arbeit nPhl p 5, rPhl p 5a wt, sowie das Histidin-Fusionsprotein rPhl p 5a wt (His). Neben anderen Molekülen wurde die IgE-Bindung der Histidin-Fusionsproteine Phl p 5a DM-Δ94-113 (His), Phl p 5a DM-Δ175-198 (His) und Phl p 5a DM-Δ94-113, 175-198 (His) sowie die des Nicht-Fusionsproteins Phl p 5a DM-Δ94-113, 175-198 vergleichend zu diesen Referenzen untersucht.

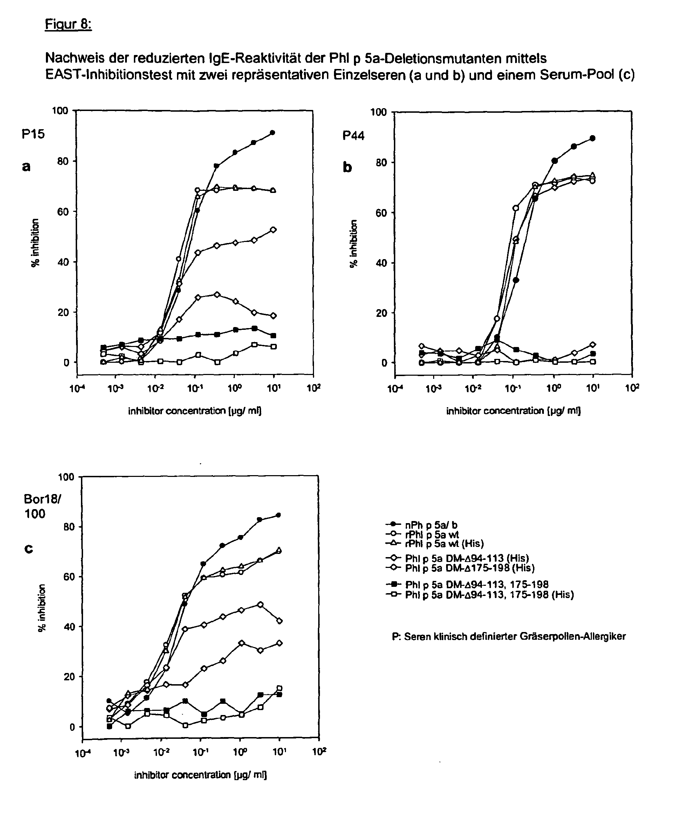

In Abb. 8a-c sind repräsentativ die spezifischen Hemmkurven von Testsubstanzen dargestellt, die mit zwei Individualseren und einem Serum-Pool von Grasspollenallergikem erhoben wurden. nPhl p 5a/b zeigte in allen Tests die stärkste Hemmwirkung (ca. 80-95% Hemmwirkung bei 10 µg/ ml Konzentation). Die Hemmwirkung von rPhl p 5a war mit 70-80% maximaler Hemmung deutlich geringer. Dieser Effekt wird durch die Zusammensetzung von nPhl p 5a/b verursacht, welches neben der Isoform Phl p 5a auch die Isoform Phl p 5b enthält. Die spezifischen IgE-Antikörper gegen Phl p 5b können durch rPhl p 5a wt nicht inhibiert werden.
Der Histidin-Fusionsanteil zeigte keinen Einfluß auf die IgE-Bindung. Dies wird durch die identischen Hemmkurven von rPhl p 5a wt (His) und rPhl p 5a wt in allen Tests deutlich. Somit ist die Gültigkeit von Tests mit Histidin-Fusionsproteinen gezeigt.

Generell konnten zwei Gruppen von Patientenseren bezüglich der qualitativen IgE-Bindung unterschieden werden.
Die erste Gruppe wird von dem Individualserum P15 repräsentiert (Abb. 8 a). Diese Allergikerseren enthielten IgE-Antikörper, deren Bindung an Phl p 5a durch beide Deletionen, Δ94-113 und Δ175-198, vermindert wurde. Die Deletionsmutante Phl p 5a DM-Δ94-113 (His) zeigte hier nur eine maximale Hemmwirkung von ca. 50% und die Deletionsmutante Phl p 5a DM-175-198 (His) eine Hemmwirkung von nur 20-30%.

Die Doppel-Deletionsmutante Phl p 5a DM-Δ94-113, 175-198 (His) konnte die Bindung von IgE-Antikörpern bei der höchsten eingesetzten Konzentration sogar nur um 0-10% inhibieren. Der Einsatz des Nicht-Fusionsprotein Phl p 5a DM-Δ94-113, 175-198 bestätigte dieses Ergebnis (0-10% maximale IgE-Hemmung).

Die zweite Gruppe von Allergikerseren, repräsentiert durch Individualserum P44 (Abb. 8 b), unterschied sich von der ersten Gruppe dadurch, dass die in den Seren enthaltenen IgE-Antikörper mit Phl p 5a DM-Δ94-113 (His) ebenso gut reagierten wie mit der Referenz rPhl p 5a wt (His) (70-80% maximale Hemmung), während keine oder nicht-nachweisbare Mengen von IgE-Antikörpern mit Phl p 5a DM-Δ175-198 (His) reagierten (0-10% maximale Hemmung).
Ebenso zeigte die Doppel-Deletionsmutante Phl p 5a DM-Δ94-113, 175-198 mit dieser Gruppe von Allergikerseren eine stark verminderte Hemmwirkung (0-10%), was sowohl für das Fusionsprotein als auch für das Fusionsanteil-freie Protein gezeigt werden konnte.
Offensichtlich enthielten die Seren dieser Allergiker IgE-Antikörper die hauptsächlich gegen Epitope des C-terminalen Teils des Moleküls gerichtet sind.

Die Messdaten der IgE-Bindungsreaktivität von IgE-Antikörpern eines Serum-Pools von 20 Allergikern unterstreichen die Wichtigkeit der Deletionen Δ94-113 und Δ175-198 für die Verminderung der IgE-Bindung des Phl p 5a (Abb. 8 c). Beide Einzel-Deletionsmutanten, Phl p 5a DM-Δ94-113 (His) und Phl p 5a DM-Δ175-198 (His) zeigen mit 40-50% bzw. ca. 30% eine geringere maximale Hemmwirkung als rPhl p 5a wt (ca. 70%). Die Doppel-Deletionsmutante Phl p 5a DM-Δ94-113, 175-198 wurde von den IgE-Antikörpern des Serum-Pools nur sehr schwach gebunden (10-15% maximale Hemmung), was übereinstimmend mit dem Test von 43 Allergikern im Streifentest auf eine stark verminderte IgE-Bindungsreaktivität dieser Phl p 5a-Variante bei sehr vielen, wenn nicht allen Grasspollen-Allergikern hinweist.

### Nachweis der Hypoallergenität der Deletionsmutanten durch Basophilen-Aktivierungstest

Mittels eines Basophilen-Aktivierungstests wurden die Auswirkungen der reduzierten IgE-Bindungsfähigkeit der Deletionsmutanten auf die funktionelle Wirkung bei der Vernetzung von membrangebundenem IgE der Effektorzellen und deren Aktivierung untersucht. Damit wurde die funktionelle Reduktion der Allergenität mit einem sensitiven In-vitro-Test gemessen.

Für den Basophilen-Aktivierungstest wird heparinisiertes Vollblut von Grasspollen-Allergikern mit verschiedenen Konzentrationen der Testsubstanzen inkubiert. Dabei können allergene Substanzen spezifische IgE-Antkörper, welche mit den hochaffinen IgE-Rezeptoren der basophilen Granulozyten assoziert, sind binden.
Die durch die Allergenmoleküle ausgelöste Vernetzung der IgE/ Rezeptor-Komplexe führt zu einer Signaltransduktion, die in der Degranulation der Effektorzellen und somit dem Auslösen der allergischen Reaktionen in vivo resultiert.

In vitro kann die Allergen-induzierte Aktivierung von basophilen Immunozyten durch Quantifizierung der Expression eines mit der Signaltransduktion der IgE-Rezeptor-Vernetzung gekoppelten Oberflächenproteins (CD203c) nachgewiesen werden (Kahlert et al., Clinical Immunology and Allergy in Medicine Proceedings of the EAACI 2002 (2003) Naples, Italy 739-744). Die Zahl der exprimierten Oberflächenproteine auf einer Zelle und der Prozentwert der aktivierten Zellen eines Zellpools wird über die Bindung eines fluoreszensmarkierten monoklonalen Antikörpers an das Oberflächenprotein und anschliessende Analyse durch Fluoreszensaktivierte Durchflusszytometrie hochsensitiv gemessen.

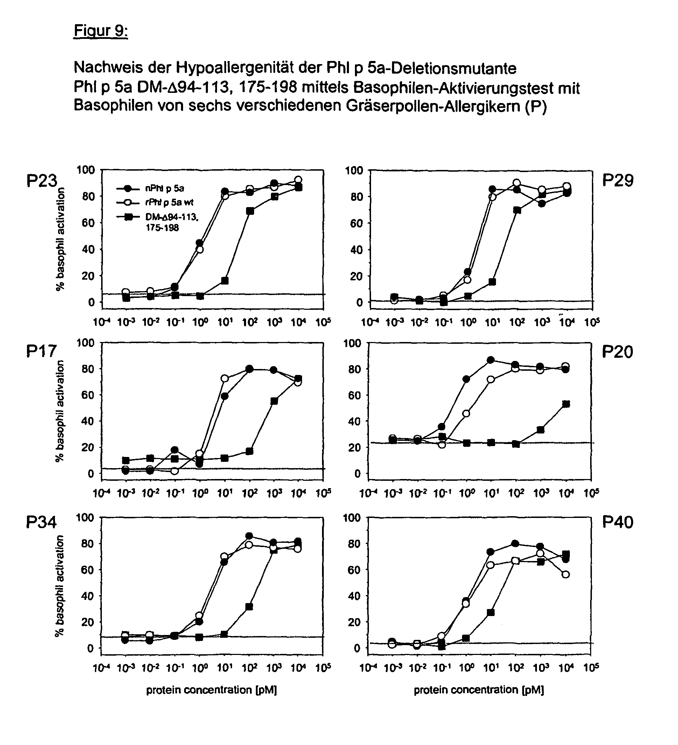

Als Referenzsubstanzen wurden hier sowohl das gereinigte natürliche Phl p 5a (nPhl p 5a) als auch rPhl p5a wt parallel zu den Testsubstanzen eingesetzt.
Die Testergebnisse der Doppel-Deletionsmutante Phl p 5a DM Δ94-113, 175-198 mit Basophilen von sechs Probanden sind in der Abbildung 9 als Kurvenverläufe dargestellt. Die Testergebnisse mit Basophilen von insgesamt 10 klinisch definierten Allergikern sind in Tabelle 2 zusammengefasst. Die A50-Werte (A50: Allergenkonzentration bei 50% der Zahl maximal aktivierter Basophilen) der Referenzmoleküle lagen indivduell variierend zwischen -1,3-15 pM für rPhl p 5a wt und ~0,3-10 pM für nPhl p 5a (Tab. 2). Dagegen lagen die A50-Werte der Deletionsvariante Phl p 5a DM Δ94-113, 175-198 zwischen ~18-8400 pM.
Aus den ermittelten A50-Werten der drei eingesetzten Substanzen konnte die allergene Wirksamkeit der Deletionsvariante Phl p 5a DM Δ94-113,175-198 in Relation zu den unveränderten Referenzmolekülen nPhl p 5a und rPhl p5a wt bei jedem Probanden ermittelt werden (Tab. 2).
Die relative allergene Wirksamkeit (Pr, Relative Potency) der Deletionsvariante Phl p 5a DM Δ94-113, 175-198 war zwischen -12-5000 fach gegenüber der Referenz rPhl p 5a wt bzw. -16-32000 fach gegenüber der Referenz nPhl p 5a erniedrigt (Tab. 2).

**Tabelle 2: Nachweis der Hypoallergenität der Deletionsmutante Phl p 5a DM-Δ94-113, 175-198 mittels Basophilen-Aktivierungstest**

| | **Testsubstanz A₅₀ [pM]^{a}** | | | **Pr-Wert^{b} Phl p 5a DM-A94-113, 175-198** | **Pr-Wert^{b} Phl p 5a DM-Δ94-113, 175-198** |
|---|---|---|---|---|---|
| **Donor^{c}** | **nPhl p 5a** | **rPhl p 5a wt** | **Phl p 5a DM-Δ94-113, 175-198** | **relativ zu rPhl p 5a wt^{d}** | **relativ zu nPhl p 5a^{e}** |
| P13 | 4,08 | 5,34 | 477,2 | 0,0111 | 0,0085 |
| P17 | 6,44 | 2,68 | 466,6 | 0,0057 | 0,0137 |
| P20 | 0,26 | 1,68 | 8433,0 | **0,0002**^{f} | **0,00003**^{f} |
| P23 | 1,02 | 1,26 | 39,2 | 0,0321 | 0,0260 |
| P24 | 1,22 | 2,57 | 58,1 | 0,0442 | 0,0209 |
| P28 | 9,43 | 11,35 | 198,2 | 0,0573 | 0,0476 |
| P29 | 1,77 | 2,34 | 33,7 | 0,0694 | 0,0525 |
| P31 | 10.15 | 14,66 | 3967,0 | 0,0037 | 0,0026 |
| P34 | 3,48 | 2,54 | 165,1 | 0,0153 | 0,0211 |
| P40 | 1,08 | 1,45 | 17,5 | **0,0829** | **0,0617** |

| | | | | | |
|---|---|---|---|---|---|
| ^{a} Allergenkonzentration bei 50 % der Zahl maximal aktivierter Basophilen ^{b} Relative Potency ^{c} Klinisch definierte Gräserpollen-Allergiker ^{d} Berechnet von A50 rPhl p 5a wt/ A50 Phl p 5a DM-Δ94-113, 175-198 ^{e} Berechnet von A50 nPhl p 5a/ A50 Phl p 5a DM-Δ94-113, 175-198 ^{f} Fett: minimale und maximale Werte | | | | | |

### T-Zell-Reaktivität

T-Helfer-Lymphozyten reagieren mit Peptidfragmenten der Allergene (ca. 12-25 Aminosäuren), die durch enzymatischen Abbau in Antigenpräsentierenden Zellen (APZ) entstehen und nach Einlagerung der geeigneten Peptide in die individuellen MHC-Klasse II-Moleküle an der Oberfläche der APZ den T-Zellen präsentiert werden. Diese allergenspezifische Aktivierung der T-Helfer-Lymphozyten ist die Voraussetzung für nachfolgende Reaktionen (Proliferation, Anergie, Apoptose) und für die funktionell Differenzierung (TH1 und TH2). Die Beeinflussung der allergenspezifischen T-Lymphozyten durch die Behandlung mit einem Allergen oder einer Allergenvariante bei der Hyposensibilisierung wird als Schlüssel für die therapeutische Wirksamkeit angesehen.

Zur Untersuchung der T-Zell-Reaktivität werden oligoklonale T-Zell-Linien (TCL) von Graminaenpollen-Allergikern unter Stimulation mit nPhl p5- oder rPhl p 5-Molekülen nach üblichen Verfahren etabliert.

In einem Proliferationstest wurden die unterschiedlichen T-Zell-Linien mit den Referenz-Allergenen nPhl p5a und rPhl p5a wt sowie der Doppel-Deletionsmutante Phl p 5a DM Δ94-113, 175-198 stimuliert. Die Proliferationsratewurde durch den Einbau von [³H]-Thymidin mit den üblichen Verfahren bestimmt.

**Tabelle 3: Nachweis der T-Zellreaktivität der Deletionsmutante Phl p 5a DM-Δ94-113, 175-198 mittels Proliferationstests mit Phl p 5-spezifischen T-Zell-Linien (TCL)**

| | | **Stimulationsindex ^{a}** | | |
|---|---|---|---|---|
| **Donor^{b}** | **TCL** | **nPhl p 5a** | **rPhl p 5a wt** | **Phl p 5a DM-Δ94-113, 175-198** |
| A | 3.2 | 9.8 | 4,9 | 4,4 |
| B | 8.2 | 21,0 | 15,5 | 13,3 |
| C | 11.2 | 5,2 | 4,7 | 7,2 |
| C | 11.3 | 3,3 | 2,9 | 3,5 |
| C | 11.43 | 3,0 | 3,9 | 2,6 |
| D | 19.1 | 6,5 | 4,7 | 7,5 |
| D | 19.2 | 9,6 | 3,3 | 2,6 |
| E | 23.22 | 21,8 | 29,0 | 20,8 |
| E | 23.50 | 7,5 | 8,4 | 6,6 |
| F | 89.23 | 1,8 | 3,5 | 1,8 |

| | | | | |
|---|---|---|---|---|
| ^{a} Berechnet von [³H]-Messwerten. cpm-Messwerte Allergen-stimulierter Zellkulturen/ cpm-Messwerte unstimulierter Zellkulturen ^{b} Donor: Klinisch definierte Gräserpollen-Allergiker | | | | |

Die Ergebnisse mit zehn TCL von sechs Allergikern zeigen, dass diese TCL durch Phl p 5a DM Δ94-113, 175-198 in vergleichbarer Stärke zur Proliferation stimuliert wurden wie durch das unveränderte natürliche bzw. das rekombinante Wildtyp-Allergen (Tab. 3).

Gegenstand der vorliegenden Erfindung sind somit Varianten des Gruppe-5-Allergens Poa p 5, welche durch eine gegenüber den bekannten Wildtyp-Allergenen verringerte IgE-Reaktivität sowie eine erhaltene Reaktivität mit T-Lymphozyten gekennzeichnet sind.

Da es sich als besonders günstig im Sinne der Erfindung herausgestellt hat, wenn bei den Gruppe-5-Allergenen Aminosäuresequenz-Bereiche fehlen bzw. entfernt werden, die den Aminosäuresequenz-Bereichen 94 - 113 und 175 - 198 des Phl p 5a entsprechen, sind insbesondere solche Allergenvarianten Gegenstand dieser Erfindung. Dabei können der erstgenannte oder der zweitgenannte Bereich einzeln, aber auch beide genannten Bereiche gleichzeitig fehlen, wobei letztere Ausführungsform ganz besonders bevorzugt ist.
Aufgrund der hohen Sequenzhomologien innerhalb der Gruppe-5-Allergene aus *Pooideae* können diese Bereiche eindeutig in Sequenzalignments der Phl p 5a-Sequenz mit Sequenzen anderer Gruppe-5-Allergene identifiziert werden. Vorzugsweise stammen die vorbeschriebenen Allergenvarianten von Phl p 5a ab bzw. entsprechen den Sequenzen gemäß SEQ ID NO 4, 6 oder 8.

Es bietet sich an, die erfindungsgemäßen Allergenvarianten, ausgehend von der klonierten DNA-Sequenz mit Hilfe gentechnischer Methoden herzustellen. Grundsätzlich kann es sich aber auch um chemische Modifikationen des nativen Allergenextrakts handeln (Fiebig, 1995, Allergo J. 4 (7), 377-382).

Naturgemäß sind über die in der vorliegenden Patentanmeldung beschriebenen Variationen von Gruppe-5-Allergenen auch weitere Veränderungen an anderen Positionen - etwa zur Erhöhung der Hypoallergenität - möglich. Bei diesen Modifikationen kann es sich beispielsweise um Aminosäure-Insertionen, -Deletionen und -Austausche, Aufspaltungen des Proteins in Fragmente sowie Fusionen des Proteins oder seiner Fragmente mit anderen Proteinen oder Peptiden handeln.

Bei der Herstellung der hier genauer beschriebenen Allergenvarianten wurde zum Zwecke einer verbesserten Reinigung der überexprimierten Proteine gentechnisch ein His-Tag eingeführt.

Gegenstand ist weiterhin ein DNA-Molekül, kodierend für eine zuvor beschriebene Allergenvariante, insbesondere entsprechend einer Sequenz gemäß SEQ ID NO 3, 5 oder 7, ein rekombinanter Expressionsvektor, enthaltend dieses DNA-Molekül sowie ein nicht humaner Wirtsorganismus, transformiert mit besagtem DNA-Molekül oder besagtem Expressionsvektor. Geeignete Wirtsorganismen können pro- oder eukaryontische, ein- oder mehrzellige Organismen wie Bakterien oder Hefen sein. Ein erfindungsgemäß bevorzugter Wirtsorganismus ist *E. coli.*

Weiterhin betrifft die Erfindung ein Verfahren zur Herstellung einer erfindungsgemäßen Allergenvariante durch Kultivieren des besagten Wirtsorganismus und Gewinnung der entsprechenden Allergenvariante aus der Kultur.

Gegenstand der vorliegenden Erfindung sind außerdem die zuvor beschriebenen Allergenvarianten, DNA-Moleküle und Expressionsvektoren in ihrer Eigenschaft als Arzneimittel.

Weiterhin sind Gegenstand der vorliegenden Erfindung pharmazeutische Zubereitungen enthaltend mindestens eine dieser Allergenvarianten bzw. ein entsprechendes DNA-Molekül oder einen entsprechenden Expressionsvektor sowie gegebenenfalls weitere Wirk- und/oder Hilfsstoffe zur Behandlung von Allergien, an deren Auslösung Gruppe-5-Allergene der *Pooideae* beteiligt sind, bzw. zur immuntherapeutischen Vakzinierung von Patienten mit Allergien, an deren Auslösung Gruppe-5-Allergene der *Pooideae* beteiligt sind und/oder zur Prävention solcher Allergien.
Handelt es sich um pharmazeutische Zubereitungen des zweiten Typs (enthaltend mindestens ein DNA-Molekül oder einen Expressionsvektor), so enthalten diese Zubereitungen vorzugsweise weiterhin Aluminiumhydroxid, ein immunstimulatorisches CpG-haltiges Oligonukleotid oder eine Kombination beider als Hilfsstoffe.

Pharmazeutische Zubereitungen im Sinne dieser Erfindung können als Therapeutika in der Human- oder Veterinärmedizin verwendet werden. Als Trägerstoffe kommen organische oder anorganische Substanzen in Frage, die sich für die parenterale Applikation eignen und mit erfindungsgemäßen Gruppe-5-Allergenvarianten nicht reagieren. Zur parenteralen Anwendung dienen insbesondere Lösungen, vorzugsweise ölige oder wässrige Lösungen, ferner Suspensionen, Emulsionen oder Implantate. Die erfindungsgemäßen Allergenvarianten können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten.
Weiterhin können durch entsprechende Formulierung der erfindungsgemäßen Allergenvarianten Depotpräparate, beispielsweise durch Adsorption an Aluminiumhydroxid, erhalten werden.

Schließlich ist Gegenstand der vorliegenden Erfindung die Verwendung mindestens einer erfindungsgemäßen Allergenvariante bzw. eines erfindungsgemäßen DNA-Moleküls oder eines erfindungsgemäßen Expressionsvektors zur Herstellung eines Arzneimittels zur Behandlung von Allergien, an deren Auslösung Gruppe-5-Allergene der *Pooideae* beteiligt sind bzw. zur immuntherapeutischen Vakzinierung von Patienten mit Allergien, an deren Auslösung Gruppe-5-Allergene der *Pooideae* beteiligt sind und/oder zur Prävention solcher Allergien.

## Patentansprüche

1. Varianten des Gruppe-5-Allergens Poa p 5, welche eine gegenüber dem bekannten Wildtyp-Allergen verringerte IgE-Reaktivität sowie eine weitgehend erhaltene Reaktivität mit T-Lymphozyten aufweisen, **dadurch gekennzeichnet, dass** ein Bereich, der dem Aminosäuresequenz-Bereich 94 - 113 des Phl p 5a entspricht, oder die Bereiche, die den Aminosäuresequenz-Bereichen 94 - 113 und 175 - 198 des Phl p 5a entsprechen, im Vergleich zu dem bekannten Wildtyp-Allergen fehlen.

2. Allergenvarianten gemäß Anspruch 1, **dadurch gekennzeichnet, dass** sie durch gentechnische Methoden rekombinant erhalten werden.

3. DNA-Molekül, kodierend für eine Allergenvariante gemäß Anspruch 1 oder 2.

4. Rekombinanter Expressionsvektor, enthaltend ein DNA-Molekül gemäß Anspruch 3, funktionell verbunden mit einer Expressionskontrollsequenz.

5. Ein nicht humaner Wirtsorganismus, transformiert mit einem DNA-Molekül gemäß Anspruch 3 oder einem Expressionsvektor gemäß Anspruch 4.

6. Verfahren zur Herstellung einer Allergenvariante gemäß Anspruch 1 oder 2, durch Kultivieren eines Wirtsorganismus gemäß Anspruch 5 und Gewinnung der entsprechenden Allergenvariante aus der Kultur.

7. Allergenvariante gemäß Anspruch 1 oder 2 als Arzneimittel.

8. Pharmazeutische Zubereitung, enthaltend mindestens eine Allergenvariante gemäß Anspruch 1 oder 2 und gegebenenfalls weitere Wirk- und/oder Hilfsstoffe zur Behandlung von Allergien, an deren Auslösung Gruppe-5-Allergene der *Pooideae* beteiligt sind.

9. Verwendung mindestens einer Allergenvariante gemäß Anspruch 1 oder 2 zur Herstellung eines Arzneimittels zur Behandlung von Allergien, an deren Auslösung Gruppe-5-Allergene der *Pooideae* beteiligt sind.

10. DNA-Molekül gemäß Anspruch 3 als Arzneimittel.

11. Rekombinanter Expressionsvektor gemäß Anspruch 4 als Arzneimittel.

12. Pharmazeutische Zubereitung, enthaltend mindestens ein DNA-Molekül gemäß Anspruch 10 oder mindestens einen Expressionsvektor gemäß Anspruch 11 und gegebenenfalls weitere Wirk- und/oder Hilfsstoffe zur immuntherapeutischen DNA-Vakzinierung von Patienten mit Allergien, an deren Auslösung Gruppe-5-Allergene der *Pooideae* beteiligt sind und/oder zur Prävention solcher Allergien.

13. Pharmazeutische Zubereitung gemäß Anspruch 12, enthaltend Aluminiumhydroxid, ein immunstimulatorisches CpG-haltiges Oligonukleotid oder eine Kombination beider als Hilfsstoffe.

14. Verwendung mindestens eines DNA-Moleküls gemäß Anspruch 10 oder mindestens eines Expressionsvektors gemäß Anspruch 11 zur Herstellung eines Arzneimittels zur immuntherapeutischen DNA-Vakzinierung von Patienten mit Allergien, an deren Auslösung Gruppe-5-Allergene der *Pooideae* beteiligt sind und/oder zur Prävention solcher Allergien.

## Claims

1. Variants of the group 5 allergen Poa p 5a, which have reduced IgE reactivity compared with the known wild-type allergen and substantially retained reactivity with T-lymphocytes, **characterised in that** a region which corresponds to amino-acid sequence region 94 - 113 of Phl p 5a or the regions which correspond to amino-acid sequence regions 94 - 113 and 175 - 198 of Phl p 5a are missing compared with the known wild-type allergens.

2. Allergen variants according to Claim 1, **characterised in that** they are obtained by recombinant genetic engineering methods.

3. DNA molecule encoding for an allergen variant according to Claim 1 or 2.

4. Recombinant expression vector containing a DNA molecule according to Claim 3, functionally connected to an expression control sequence.

5. Non-human host organism transformed with a DNA molecule according to Claim 3 or an expression vector according to Claim 4.

6. Process for the preparation of an allergen variant according to Claim 1 or 2 by cultivation of a host organism according to Claim 5 and isolation of the corresponding allergen variant from the culture.

7. Allergen variant according to Claim 1 or 2 as medicament.

8. Pharmaceutical composition comprising at least one allergen variant according to Claim 1 or 2 and optionally further active compounds and/or adjuvants for the treatment of allergies in the triggering of which group 5 allergens of the *Pooideae* are involved.

9. Use of at least one allergen variant according to Claim 1 or 2 for the preparation of a medicament for the treatment of allergies in the triggering of which group 5 allergens of the *Pooideae* are involved.

10. DNA molecule according to Claim 3 as medicament.

11. Recombinant expression vector according to Claim 4 as medicament.

12. Pharmaceutical composition comprising at least one DNA molecule according to Claim 10 or at least one expression vector according to Claim 11 and optionally further active compounds and/or adjuvants for the immunotherapeutic DNA vaccination of patients having allergies in the triggering of which group 5 allergens of the *Pooideae* are involved and/or for the prevention of such allergies.

13. Pharmaceutical composition according to Claim 12, comprising aluminium hydroxide, an immunostimulatory CpG-containing oligonucleotide or a combination of the two as adjuvants.

14. Use of at least one DNA molecule according to Claim 10 or at least one expression vector according to Claim 11 for the preparation of a medicament for the immunotherapeutic DNA vaccination of patients having allergies in the triggering of which group 5 allergens of the *Pooideae* are involved and/or for the prevention of such allergies.

## Revendications

1. Variantes de l'allergène de groupe 5 Poa p 5a, qui ont une réactivité IgE réduite par comparaison avec l'allergène de type sauvage connu et une réactivité substantiellement conservée avec les lymphocytes T, **caractérisées en ce qu'**une région qui correspond à la région de séquence d'acides aminés 94 - 113 de Phl p 5a ou les régions qui correspondent aux région de séquence d'acides aminés 94 - 113 et 175 - 198 de Phl p 5a sont manquantes par comparaison avec les allergènes de type sauvage connus.

2. Variantes d'allergène selon la revendication 1, **caractérisées en ce qu'**elles sont obtenues par des méthodes de génie génétique recombinant.

3. Molécule d'ADN codant pour une variante d'allergène selon la revendication 1 ou 2.

4. Vecteur d'expression recombinant contenant une molécule d'ADN selon la revendication 3, lié fonctionnellement à une séquence de contrôle d'expression.

5. Organisme hôte non humain transformé avec une molécule d'ADN selon la revendication 3 ou un vecteur d'expression selon la revendication 4.

6. Procédé pour la préparation d'une variante d'allergène selon la revendication 1 ou 2 par mise en culture d'un organisme hôte selon la revendication 5 et par isolation de la variante d'allergène correspondante vis-à-vis de la culture.

7. Variante d'allergène selon la revendication 1 ou 2 comme médicament.

8. Composition pharmaceutique comprenant au moins une variante d'allergène selon la revendication 1 ou 2 et optionnellement d'autres composés actifs et/ou adjuvants pour le traitement d'allergies dans la sélection de quel groupe 5 des allergènes de *Pooideae* sont impliqués.

9. Utilisation d'au moins une variante d'allergène selon la revendication 1 ou 2 pour la préparation d'un médicament pour le traitement d'allergies dans la sélection de quel groupe 5 des allergènes de *Pooideae* sont impliqués.

10. Molécule d'ADN selon la revendication 3 comme médicament.

11. Vecteur d'expression recombinant selon la revendication 4 comme médicament.

12. Composition pharmaceutique comprenant au moins une molécule d'ADN selon la revendication 10 ou au moins un vecteur d'expression selon la revendication 11 et optionnellement d'autres composés actifs et/ou adjuvants pour la vaccination par ADN immunothérapeutique de patients ayant des allergies dans la sélection de quel groupe 5 des allergènes de *Pooideae* sont impliqués et/ou pour la prévention de telles allergies.

13. Composition pharmaceutique selon la revendication 12, comprenant hydroxyde d'aluminium, un oligonucléotide contenant CpG immunostimulatoire ou une combinaison des deux en tant qu'adjuvants.

14. Utilisation d'au moins une molécule d'ADN selon la revendication 10 ou d'au moins un vecteur d'expression selon la revendication 11 pour la préparation d'un médicament pour la vaccination par ADN immunothérapeutique de patients ayant des allergies dans la sélection de quel groupe 5 des allergènes de *Pooideae* sont impliqués et/ou pour la prévention de telles allergies.
